# EUROPÄISCHE PATENTANMELDUNG

(11) **EP 1 004 579 A1**
(43) Veröffentlichungstag der Anmeldung: **31.05.2000**
(21) Anmeldenummer: 99122280.3
(22) Anmeldetag: 09.11.1999
(51) Int. Cl.: C07D 209/08

(54) **Verfahren zur Herstellung von 2,3-Dihydroindolen (Indolinen), neuartige 2,3-Dihydroindole sowie deren Verwendung**

(30) Priorität: 20.11.1998 DE 19853558
(71) Anmelder: Aventis Research & Technologies GmbH & Co KG, 65926 Frankfurt am Main (DE)
(72) Erfinder: Beller, Matthias, Prof., Dr., 18119 Rostock (DE); Riermeier, Thomas, Dr., 65439 Flörsheim (DE); Trauthwein, Harald, Dr., 80636 München (DE); Breindl, Claudia, 80333 München (DE)

(57) **Zusammenfassung**

Verfahren zur Herstellung von 2,3-Dihydroindolen der allgemeinen Formel (I) 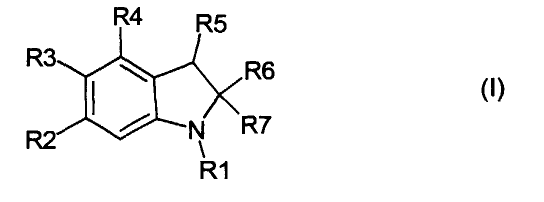 durch Umsetzung von Halogenstyrolen der allgemeinen Formel (IIa) oder (IIb) 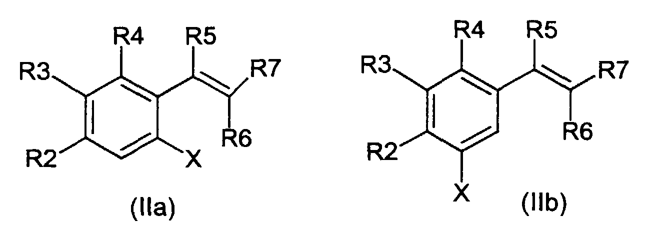 mit Aminen der allgemeinen Formel (III)

R¹-NH₂ (III)

in mindestens einem inerten Lösungsmittel oder in Wasser sowie in Gegenwart mindestens einer Base.

## Beschreibung

Eine Vielzahl von Indolderivaten, insbesondere von Indolalkaloiden, werden als Medikamente in der Human- und Tiermedizin eingesetzt (E. Breitmaier "Alkaloide: Betäubungsmittel, Halluzinogene und andere Wirkstoffe", Teubner Studienbücher Chemie, 1997). 2,3-Dihydroindolderivate sind gleichfalls als Wirkstoffe von großem pharmakologischen Interesse. Darüber hinaus dienen sie als Ausgangsmaterialien für entsprechende Indole, da sie sehr einfach dehydriert werden können (vgl. B. Robinson, Chem. Rev. 1969, 69, 785; sowie Y. Kikugawa, M. Kashimura, Synthesis 1982, 785).

Häufig angewandte Methoden zur Synthese von Indolen und 2,3-Dihydroindolen sind im universitären Bereich die Fischer'sche Indolsynthese (B. Robinson, Chem. Rev. 1969, 69, 227) und die Reissert-Reaktion (G. Blasko, P. Kerekes, S. Makleit, Alkaloids (Academic Press) 1987, 31, 1-28; J. G. Cannon, B. J. Demopoulos, J. P. Long, J. R. Flynn, F. M. Sharabi, J. Med. Chem. 1981, 238-40). Die mehrstufigen Reaktionssequenzen sind nur aufwendig durchzuführen und deshalb industriell unbedeutend.

Alternativ dazu ist die Cyclisierung von halogensubstituierten N-(2-Arylethyl)aminen via Arin-Intermediate beschrieben (R. Huisgen, H. König, Chem. Ber. 1959, 92, 203; R. Huisgen, H. König, N. Bleeker, Chem. Ber, 1959, 92, 424; H. König, R. Huisgen, Chem. Ber. 1959, 92, 429; H. Iida, S. Aoyagi, C. Kibayashi, J. Chem. Soc., Perkin I 1975, 2502). Als Base wird in diesen Berichten die extrem reaktive und industriell schwierig handhabbare Verbindung Phenyllithium eingesetzt. Die Cyclisierung gelingt nur mit Ausbeuten unter 50%. Da die als Edukte verwendeten 2-Halophenylethylaminen nur durch eine komplizierte mehrstufige Synthese erhalten werden können (Gesamtausbeuten 13 % bzw. 18 %), findet auch diese Methode keine breite Anwendung.

Aufgabe der vorliegenden Erfindung ist die Bereitstellung eines neuen Herstellungsverfahrens für 2,3-Dihydroindolderivate, das nicht die Nachteile der bekannten Verfahren zeigt und darüber hinaus für die Durchführung im Industriemaßstab geeignet ist. Eine weitere Aufgabe der vorliegenden Erfindung ist die Bereitstellung neuartiger 2,3-Dihydroindole sowie deren Verwendung.

Diese Aufgabe wird gelöst durch ein Verfahren zur Herstellung von 2,3-Dihydroindolen der allgemeinen Formel (I) 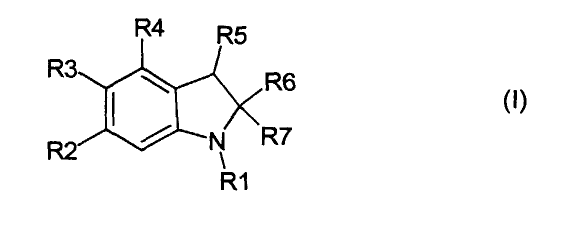 in der die Reste R1 bis R7 unabhängig voneinander aus der Gruppe von Wasserstoff, Fluor, Chlor, (C₁-C₈)-Alkyl, O-Alkyl-(C₁-C₈), OCO-Alkyl-(C₁-C₈), OH, NO₂, Si(Alkyl)₃-(C₁-C₈), CF₃, CN, COOH, CHO, SO₃H, NH₂, NH-Alkyl-(C₁-C₈), N-Alkyl₂-(C₁-C₈), NH-Ar, NAr₂, P-Alkyl₂-(C₁-C₈), SO₂-Alkyl-(C₁-C₆), SO-Alkyl-(C₁-C₆), NHCO-Alkyl-(C₁-C₄), COO-Alkyl-(C₁-C₈), CONH₂, CONH-Alkyl-(C₁-C₈), CO-Alkyl-(C₁-C₈), NHCOH, NHCOO-Alkyl-(C₁-C₄), CHCH-CO₂-Alkyl-(C₁-C₈), CHCHCO₂H, P-Alkyl₂-(C₁-C₈), POAlkyl₂-(C₁-C₄), PO₃H₂, PO(O-Alkyl-(C₁-C₆))₂, SO₃-Alkyl-(C₁-C₄), SO₂-Alkyl-(C₁-C₆), SO-Alkyl-(C₁-C₆), Si(Alkyl)₃-(C₁-C₈), Ar, O-Ar, CO-Ar, COO-Ar, PO-Ar₂ und PAr₂; wobei Ar
- einen aromatischen Rest mit bis zu 14 Kohlenstoffatomen; oder
- einen Heteroaromaten darstellt, ausgewählt aus der Gruppe der fünf-, sechs- oder siebengliedrigen Ringe mit mindestens einem Stickstoff-, Sauerstoff- und/oder Schwefelatom im Ring;
durch Umsetzung von Halogenstyrolen der allgemeinen Formel (IIa) oder (IIb) 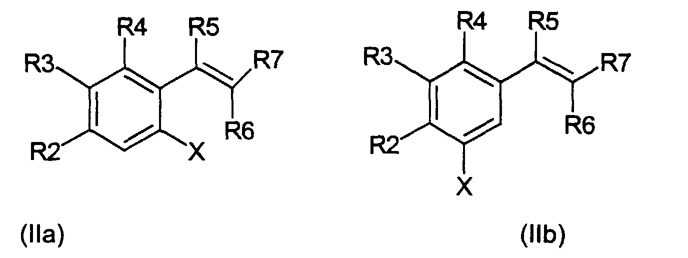 mit Aminen der allgemeinen Formel (III)

R¹-NH₂ (III)

wobei in den Formeln (IIa), (IIb) und (III)
- R¹ bis R⁷ die gleiche Bedeutung haben wie in Formel (I);
- X ausgewählt ist aus der Gruppe von Chlor, Brom, Iod, OSO₂CF₃, OSO₂Aryl-(C₆-C₁₀), OSO₂Alkyl-(C₁-C₈) und N₂⁺Y⁻, wobei Y ein Chlor-, Brom- oder Iodatom oder ein Tetrafluoroborat- oder Tetraphenylboratanion darstellt;
in mindestens einem inerten Lösungsmittel oder in Wasser sowie in Gegenwart mindestens einer Base, ausgewählt aus der Gruppe von
- primären, sekundären und tertiären Alkoxiden;
- primären und sekundären Amiden von Alkali- und/oder Erdalkalielementen;
- Alkyl- und Arylverbindungen von Alkali und/oder Erdalkalimetallen; und
- Carbonaten, Hydroxiden, Hydrogencarbonaten von Lithium, Natrium, Kalium, Calcium, Magnesium und Cäsium.

Der Rest R¹ kann gemäß einer bevorzugten Ausführungsform einen (C₁-C₈)-Alkyl-, Phenyl-, Naphthyl-, Anthryl-, Phenanthryl-, Biphenyl-, Pyridin-, Pyrimidin-, Oxazol-, Imidazol-, Pyrazin-, Chinolin-, Indol-, Furan-, Benzofuran- oder Thiophenrest darstellen.

Die Reste R² bis R⁷ können entsprechend einer besonders bevorzugten Ausführungsform der Erfindung unabhängig voneinander ausgewählt sein aus der Gruppe von Wasserstoff, (C₁-C₈)-Alkyl, O-Alkyl-(C₁-C₈), O-CO-Alkyl-(C₁-C₈), N-Alkyl₂-(C₁-C₈), Ar, F, Cl, NO₂, CN, COOH, CHO, SO₂-Alkyl-(C₁-C₄), NH-Alkyl-(C₁-C₈), NH-Ar, NAr₂, COO-Alkyl-(C₁-C₈), CONH₂, CONH-Alkyl-(C₁-C₈), CO-(C₁-C₈)-Alkyl, CO-Ar und/oder PO-Ar₂; wobei Ar die gleiche Bedeutung hat wie zuvor beschrieben und insbesondere einen Phenylrest darstellt.

Ebenfalls können die zuvor genannten Reste substituiert sein. Besonders bevorzugt sind die Fälle, in denen
- der aromatische Rest Ar bis zu 5 Substituenten;
- der Heteroaromat bis zu 4 Substituenten; und/oder
- der Rest R¹ bis zu 5 Substituenten
aufweisen, die unabhängig voneinander ausgewählt sind aus der Gruppe von Fluor, Chlor, CF₃, OH, NO₂, CN, R⁵, O-R⁵, CHO, CO-R⁵, COOH, COO-R⁵, OCO-R⁵, SiR⁵₃, NH₂, NH-R⁵, N-R⁵₂, SO-R⁵, SO₂-R⁵, SO₃H, SO₃-R⁵, CONH₂, NHCOH, NHCO-R⁵, NHCOO-R⁵, CHCH-CO₂-Alkyl-(C₁-C₈), PO-R⁵₂, P-R⁵₂, PO₃H₂, PO(O-Alkyl-(C₁-C₆))₂ und CHCHCO₂H; wobei R⁵ einen Alkylrest mit 1 bis 8 Kohlenstoffatomen oder einen Arylrest Ar darstellt; wobei Ar die gleiche Bedeutung hat wie in Anspruch 1 und insbesondere einen Phenylrest darstellt.

Das erfindungsgemäße Verfahren eignet sich insbesondere zur Herstellung von Dihydroindolen, in denen der Rest R¹ einen 4-Fluorphenyl- oder 2-Methoxyphenylrest darstellt.

Sofern der Rest Ar einen Heteroaromaten darstellt, können an diesen heteroaromatischen Ring bis zu vier weitere aromatische, heteroaromatische und/oder aliphatische Ringe ankondensiert sein.

Die für das Verfahren notwendigen Ausgangsmaterialien wie Amine und 2- oder 3-Halostyrole sind kommerziell verfügbar. Spezielle substituierte Halostyrole können einfach durch die palladiumkatalysierte Heck-Reaktion oder Wittig-Reaktion dargestellt werden, so daß für das erfindungsgemäße Verfahren eine Vielzahl an Substraten zur Verfügung steht.

Angesichts der allgemein bekannten Tatsache, demnach Arincyclisierungen von 2-Halophenylethylaminen in nur mäßiger Ausbeute ablaufen (R. Huisgen, H. König, Chem. Ber. 1959, 92, 203; R. Huisgen, H. König, N. Bleeker, Chem. Ber, 1959, 92, 424; H. König, R. Huisgen, Chem. Ber. 1959, 92, 429; H. Iida, S. Aoyagi, C. Kibayashi, J. Chem. Soc., Perkin I 1975, 2502), war es überraschend, daß entsprechend dem erfindungsgemäßen Verfahren beliebig substituierte 2,3-Dihydroindole in nur einer Stufe ausgehend von Halostyrolderivaten und primären Aminen in guter Ausbeute erhalten werden können.

Die Base kann bei dem Verfahren ausgewählt sein aus der Gruppe von Kaliumalkoxiden, Cäsiumalkoxiden, Alkaliamiden, Lithiumorganylen und Magnesiumorganylen.
Bevorzugterweise ist die Base ausgewählt aus der Gruppe von Kalium-tert.-butanolat, Kaliumisopropanolat, Natriummethanolat, Kaliummethanolat, Natriumethanolat, Magnesiummethanolat, Calciumethanolat, Lithiumdiisopropylamid, Lithiumdiethylamid, Natriumdimethylamid, Cäsiumcarbonat, Butyllithium, Phenyllithium, Phenylmagnesiumchlorid und Kaliumhydroxid.

Das inerte Lösungsmittel kann ausgewählt sein aus der Gruppe von aromatischen oder aliphatischen Kohlenwasserstoffen, Estem, Aminen und Amiden. Bevorzugterweise ist das Lösungsmittel ausgewählt aus der Gruppe von THF (Tetrahydrofuran), Dioxan, Diethylether, Diglyme, MTBE (Methyl-tert.-butylether), DME (Dimethylether), Toluol, Xylolen, Anisol, Ethylacetat, Ethylencarbonat und Propylencarbonat.

Bei dem erfindungsgemäßen Verfahren wird die Base im allgemeinen in einer Menge von 0,5 bis 10 Equivalenten, bezogen auf das Halogenstyrol der Formel (IIa) oder (IIb), eingesetzt.
Vorzugsweise wird die Base in einer Menge von 0,8 bis 5 Equivalenten, bezogen auf das Halogenstyrol der Formel (IIa) oder (IIb), eingesetzt.

Das Verfahren entsprechend der Erfindung wird üblicherweise bei einer Temperatur zwischen 0 und 200 °C, vorzugsweise zwischen 40 und 180 °C, insbesondere zwischen 60 und 160 °C, durchgeführt.

Zur Herstellung von Indolen entsprechend der vorliegenden Erfindung wird die zuvor beschriebene Umsetzung in Gegenwart eines zusätzlichen Dehydrierungsmittels durchgeführt.
Ebenso ist es möglich, daß nach erfolgter Umsetzung zu der Reaktionsmischung ein Dehydrierungsmittel hinzugegeben wird.
Das Dehydrierungsmittel ist bevorzugt ausgewählt aus der Gruppe von Sauerstoff, Schwefel und DDQ (2,3-Dichlor-3,4-dicyanochinon).

Die Umsetzung wird im allgemeinen drucklos gefahren, es kann jedoch auch bei Drücken bis zu 100 bar gearbeitet werden.

Mit dem erfindungsgemäßen Verfahren ist es erstmals gelungen, neuartige 2,3-Dihydroindole herzustellen, ausgewählt aus der Gruppe von N-(2-Methoxyphenyl)-2,3-dihydroindol, N-(4-Fluorphenyl)-4-chlor-2,3-dihydroindol, N-(4-Fluorphenyl)-2,3-dihydroindol und N-(3-Ethoxypropyl)-2,3-dihydroindol.
Diese neuen Dihydroindole können insbesondere verwendet werden als
- Herbizid;
- Vorstufe und/oder Intermediat für die Herstellung von Farbstoffen, Aminosäuren, photosensitives und/oder thermosensitives Material, Kosmetika;
- Antioxidans für Kosmetika (im Haut- und Haarpflegebereich) und Zahnpflegeprodukte;
- Angiotensin(II)-Antagonist;
- Lipoxygenase-Inhibitor;
- Kaliumkanal-Blocker, insbesondere bei der Behandlung der Alzheimer-Krankheit oder von Depressionen;
- Inhibitor für Enzyme, insbesondere Protein-Kinase;
- Polymerisationskatalysator, insbesondere bei der Herstellung von Polyestern;
- Stabilisator für Kunststoffe, insbesondere für PVC oder Polyamide;
- Ligand für Metallkatalysatoren;
- flammenhemmendes Additiv; und
- Antistatikum in Kunststoffen.

Die nachstehenden Beispiele dienen der Erläuterung der Erfindung, ohne diese zu beschränken.

### Beispiele

### Allgemeines

Die verwendeten absoluten Lösemittel wurden über Molekularsieb 4 Å von der Fa. Fluka bezogen und unter Argon gelagert. Die benötigten Amine wurden über CaH₂ destilliert und unter Argonatmosphäre gelagert. Die in diesen Reaktionen verwendeten Chloraromaten wurden mehrmals entgast, mit Argon gesättigt und über ein Molekularsieb 4 Å gelagert. Für die Extraktion und säulenchromatographische Reinigung von Reaktionsprodukten wurden technische Lösemittel verwendet, welche vor Gebrauch destilliert wurden. Alle Reaktionen wurden unter Ausschluß von Luft und Wasser unter Argon-Atmosphäre in einem 38 ml Ace-Druckrohr (von der Fa. Aldrich) durchgeführt.

### a) Allgemeine Arbeitsvorschrift für Hydroaminierungen und Arin-Cyclisierungs-Reaktionen mit aromatischen Aminen (im folgenden AAV1):

In einem Druckrohr werden unter Schutzgasatmosphäre 2,0 mmol des Halogenstyrols zusammen mit derjeweiligen Menge des Amins in 10 ml Toluol gelöst. Nach Zugabe der Base wird das verschlossene Reaktionsgefäß in ein auf 135 °C erhitztes Ölbad gegeben und die Reaktionsmischung gut gerührt. Nach 36 h läßt man die Reaktionsmischung auf Raumtemperatur abkühlen und gibt unter Rühren 20 ml Wasser zu. Die wäßrige Phase wird dreimal mit Methylenchlorid extrahiert. Nachdem die vereinigten organischen Phasen über Magnesiumsulfat getrocknet wurden, wird das Lösemittel im Vakuum entfernt. Das so erhaltene Rohprodukt wird mit dem angegebenen Laufmittel säulenchromatographisch gereinigt und anhand von NMR- und massenspektroskopischen Untersuchungen charakterisiert. Die Numerierung der Kohlenstoffatome in den Formeln bezieht sich auf die NMR-Zuordnung.

### Beispiel 1 (N-Phenyl-2,3-dihydroindol):

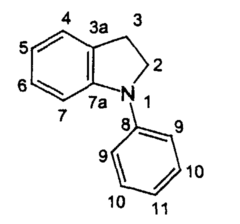

- Edukte:: 0,28 g (2,0 mmol) 2-Chlorstyrol, 0,28 g (3,0 mmol) Anilin, 0,67 g (6,0 mmol) Kalium-tert.-butylat.
- Laufmittel:: Hexan.
- Ausbeute:: 0,21 g (53 % der Theorie).

^{**1**}**H-NMR** (360 MHz, 25 °C, CDCl₃): δ = 7,32 (dd, 3JHH = 8,0 Hz, 3JHH = 7,1 Hz, 2H, 10-H), 7,21 (d, ³*J*_{HH} = 8,0 Hz, 2H, 9-H), 7,14(d, ³*J*_{HH} = 7,1 Hz, 1H, 4-H), 7,12 (d, ³*J*_{HH} = 8,0 Hz, 1H, 7-H), 7,05 (dd, ³*J*_{HH} = 8,0 Hz, ³*J*_{HH} = 7,5 Hz, 1H, 6-H), 6,94 (t, ³*J*_{HH} = 7,1 Hz, 1H, 11-H), 6,73 (dd, ³*J*_{HH} = 7,5 Hz, ³*J*_{HH} = 7,1Hz, 1H, 5-H), 3,92 (t, ³*J*_{HH} = 8,4 Hz, 2H, 2-H), 3,10 (t, ³*J*_{HH} = 8,4 Hz, 2H, 3-H).
^{**13**}**C{**^{**1**}**H}-NMR** (90 MHz, 25 °C, CDCl₃): δ = 147,1 (C8), 144,2 (C7a), 131,2 (C3a), 129,1 (C10), 127,1 (C4), 125,0 (C6), 120,9 (C5), 118,8 (C7), 117,7 (C9), 108,2 (C11), 52,1 (C2), 28,2 (C3).
**MS** (70 eV): *m/z* = 195 (M⁺), 165, 116, 91, 77.

### Beispiel 2 (N-(4-Fluorphenyl)-2,3-dihydroindol):

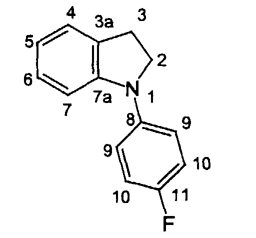

- Edukte:: 0,28 g (2,0 mmol) 2-Chlorstyrol, 0,33 g (3,0 mmol) 4-Fluoranilin, 0,67 g (6,0 mmol) Kalium-tert.-butylat.
- Laufmittel:: Hexan
- Ausbeute:: 0,23 g (54 % der Theorie).

^{**1**}**H-NMR** (360 MHz, 25 °C, CDCl₃): δ = 7,19 - 6,95 (m, 7H, HAr), 6,73 (dd, ³*J*_{HH} = 7,5 Hz, ³*J*_{HH} = 7,1 Hz, 1H, 5-H), 3,85 (t, ³*J*_{HH} = 8,4 Hz, 2H, 2-H), 3,08 (t, ³*J*_{HH} = 8,4 Hz, 2H, 3-H).
^{**13**}**C{**^{**1**}**H}-NMR** (90 MHz, 25°C, CDCl₃): δ = 157,7 (d, ¹*J*_{CF} = 241,1 Hz, C11), 147,6 (C7a), 140,51 (C8), 130,9 (C3a), 127,1 (C4), 125,0 (C6), 119,8 (d, ³*J*_{CF} = 8,4 Hz, C9), 118,8 (C5), 115,7 (d, ²*J*_{CF} = 21,1 Hz C10), 107,6 (C7), 52,7 (C2), 28,2 (C3).
**MS** (70 eV): *m/z* = 213 (M⁺), 183, 165, 116, 105,91.

### Beispiel 3 (N-(2-Methoxyphenyl)-2,3-dihydroindol):

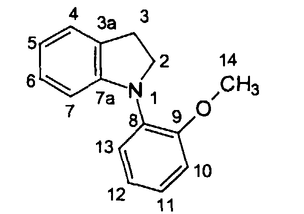

- Edukte:: 0,28 g (2,0 mmol) 2-Chlorstyrol, 0,37 g (3,0 mmol) o-Anisidin, 0,67 g (6,0 mmol) Kalium-tert.-butylat.
- Laufmittel:: (Essigester / Hexan = 1 / 50).
- Ausbeute:: 0,26 g (58 % der Theorie).

^{**1**}**H-NMR** (360 MHz, 25 °C, CDCl₃): δ = 7,35 (d, ³*J*_{HH} = 7,5 Hz, 13-H), 7,15 - 7,11 (m, 2H, 4-H/7-H), 7,10 - 9,90 (m, 3H, 5-H/6-H/12-H), 6,68 (dd, ³*J*_{HH} = 8,0 Hz, ³*J*_{HH} = 7,5 Hz, 1H, 11-H), 6,46 (d, ³*J*_{HH} = 8,0 Hz, 10-H), 3,88 (t, ³*J*_{HH} = 8,4 Hz, 2H, 2-H), 3,84 (s, 3H, 14-H), 3,14 (t, ³*J*_{HH} = 8,4 Hz, 2H, 3-H).
^{**13**}**C{**^{**1**}**H}-NMR** (90 MHz, 25 °C, CDCl₃): δ = 154,5 (C9), 149,6 (C7a), 134,0 (C3a), 130,2 (C8), 126,9 (CH), 125,3 (CH), 124,6 (CH), 123,3 (CH), 121,0 (CH), 118,0 (CH), 112,3 (CH), 109,0 (CH), 55,5 (C14), 53,5 (C2), 28,8 (C3).
**MS** (70 eV): *m/z* = 225 (M⁺), 210(M⁺-CH₃), 194, 180, 165, 152.

### Beispiel 4 (N-(4-Fluorphenyl)-4-chlor-2,3-dihydroindol):

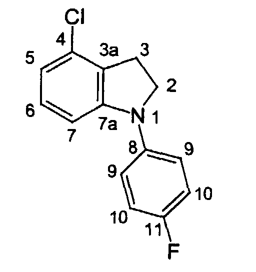

- Edukte:: 0,35 g (2,0 mmol) 2,6-Dichlorstyrol, 0,28 g (2,5 mmol) 4-Fluoranilin, 0,34 g (3,0 mmol) Kalium-tert.-butylat.
- Laufmittel:: Hexan.
- Ausbeute:: 0,26 g (50 % der Theorie).

^{**1**}**H-NMR** (360 MHz, 25 °C, CDCl₃): δ = 7,12 - 6,64 (m, 7H, HAr), 3,88 (t, ³*J*_{HH} = 8,4 Hz, 2H, 2-H), 3,10 (t, ³*J*_{HH} = 8,4 Hz, 2H, 3-H).
^{**13**}**C{**^{**1**}**H}-NMR** (90 MHz, 25 °C, CDCl₃): δ = 157,8 (d, ¹*J*_{CF} = 241,2 Hz, C11), 149,1 (C7a), 140,0 (C8), 131,0 (C4), 128,9 (C3a), 128,7 (C6), 120,4 (d, ³*J*_{CF} = 6,9 Hz, C9), 118,7 (C5), 115,9 (d, ²*J*_{CF} = 22,1 Hz, C10), 105,7 (C7), 52, 5 (C2), 27,5 (C3).
**MS** (70 eV): *m/z* = 247 (M⁺), 211 (M⁺-HCl), 183, 105, 89.

### b) Allgemeine Arbeitsvorschrift für Hydroaminierungen und Arin-Cyclisierungs-Reaktionen mit aliphatischen Aminen (im folgenden AAV2):

Unter Schutzgasatmosphäre werden in einem 38 ml Ace-Druckrohr (von der Fa. Aldrich) 6,0 mmol Amin in 12 ml THF gelöst und die Lösung auf-78°C abgekühlt. Nach Zugabe von 10 mol% n-BuLi-Lösung werden 4,0 mmol 3-Chlorstyrol zugesetzt und das verschlossene Reaktionsgefäß bei -30°C 4 h gerührt, bevor man auf Raumtemperatur erwärmen läßt. Anschließend wird das Lösemittel im Vakuum entfernt, der Rückstand in 12 ml Toluol aufgenommen, mit angegebener Menge Kalium-tert-butylat versetzt und in ein auf 135 °C vorgeheiztes Ölbad gegeben. Die Reaktionsmischung wird gut gerührt und nach 36 h läßt man auf Raumtemperatur abkühlen und gibt unter Rühren 20 ml Wasser zu. Die wäßrige Phase wird dreimal mit Methylenchlorid extrahiert. Nachdem die vereinigten organischen Phasen über Magnesiumsulfat getrocknet wurden, wird das Lösemittel im Vakuum entfernt. Das so erhaltene Rohprodukt wird mit dem angegebenen Laufmittel säulenchromatographisch gereinigt.

### Beispiel 5 (N-Butyl-2,3-dihydroindol):

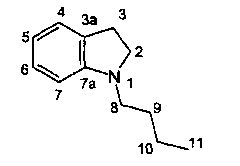

- Edukte:: 0,55 g (4,0 mmol, 0,51 ml) 3-Chlorstyrol, 0,44 g (6,0 mmol, 0,60 ml) n-Butylamin, 10 mol% n-BuLi-Lösung (1,6 m in Hexan), 1,35 g (12,0 mmol) Kalium-tert.-butylat.
- Laufmittel:: Hexan / Essigester (5:1).
- Ausbeute:: 53 % d. Th.

^{**1**}**H-NMR** (400 MHz, 25 °C, CDCl₃): δ = 6,94 (m, 2H, 4-H, 6-H), 6,53 (t, ³*J*_{HH} = 7,5 Hz, 1H, 5-H), 6,38 (d, ³*J*_{HH} = 8,0 Hz, 1H, 7-H), 3,23 (t, ³*J*_{HH} = 7,5 Hz, 2H, 2-H), 2,96 (t, ³*J*_{HH} = 7,5 Hz, 2H, 3-H), 2,84 (t, ³*J*_{HH} = 7,5 Hz, 2H, 8-H), 1,52 (q, ³*J*_{HH} = 7,5 Hz, 2H, 9-H), 1,31 (Sextett, ³*J*_{HH} = 7,5 Hz, 2H, 10-H), 0,96 (t, ³*J*_{HH} = 7,5 Hz, 3H, 11-H).
^{**13**}**C{**^{**1**}**H}-NMR** (100 MHz, 25 °C, CDCl₃): δ = 152,1 (C7a), 129,3 (C3a), 127,7 (C4), 126,6 (C6), 116,5 (C5), 106,2 (C7), 52,4 (C2), 48,3 (C8), 27,9 (C3), 22,0 (C9), 19,8 (C10), 13,3 (C11).
**MS** (70 eV): *m/z* = 175 (M⁺), 132 (M⁺ - C₃H₇), 117, 91, 77.

### Beispiel 6 (N-(3-Ethoxypropyl)-2,3-dihydroindol):

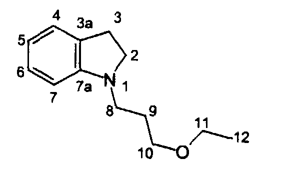

- Edukte:: 0,55 g (4,0 mmol, 0,51 ml) 3-Chlorstyrol, 0,62 g (6,0 mmol, 0,72 ml) 3-Ethoxypropylamin, 10 mol% n-BuLi-Lösung (1,6 m in Hexan), 1,35 g (12,0 mmol) Kalium-tert.-butylat.
- Laufmittel:: Hexan / Essigester (5:1).
- Ausbeute:: 53 % d. Th.

^{**1**}**H-NMR** (400 MHz, 25 °C, CDCl₃): δ = 6,94 (m, 2H, 4-H, 6-H), 6,53 (t, ³*J*_{HH} = 7,5 Hz, 1H, 5-H), 6,38 (d, ³*J*_{HH} = 8,0 Hz, 1H, 7-H), 3,23 (t, ³*J*_{HH} = 7,5 Hz, 2H, 2-H), 2,96 (t, ³*J*_{HH} = 7,5 Hz, 2H, 3-H), 2,84 (t, ³*J*_{HH} = 7,5 Hz, 2H, 8-H), 1,52 (q, ³*J*_{HH} = 7,5 Hz, 2H, 9-H), 1,31 (Sextett, ³*J*_{HH} = 7,5 Hz, 2H, 10-H), 0,96 (t, ³*J*_{HH} = 7,5 Hz, 3H, 11-H),
^{**13**}**C{**^{**1**}**H}-NMR** (100 MHz, 25 °C, CDCl₃): δ = 152,1 (C7a), 129,3 (C3a), 127,7 (C4), 126,6 (C6), 116,5 (C5), 106,2 (C7), 52,4 (C2), 48,3 (C8), 27,9 (C3), 22,0 (C9), 19,8 (C10), 13,3 (C11).
**MS** (70 eV): *m/z* = 205 (M⁺), 132 (M⁺ - CH₂CH₂-OEt), 117, 103, 91, 77.

### Beispiel 7 (N-(2-Phenylethyl)-2,3-dihydroindol):

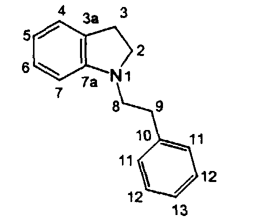

- Edukte:: 0,55 g (4,0 mmol, 0,51 ml) 3-Chlorstyrol, 0,73 g (6,0 mmol, 0,76 ml) 2-Phenylethylamin, 10 mol% n-BuLi-Lösung (1,6 m in Hexan), 1,35 g (12,0 mmol) Kalium-tert.-butylat.
- Laufmittel:: Hexan / Essigester (5:1).
- Ausbeute:: 53 % d. Th.

^{**1**}**H-NMR** (400 MHz, 25 °C, CDCl₃): δ = 6,94 (m, 2H, 4-H, 6-H), 6,53 (t, ³*J*_{HH} = 7,5 Hz, 1H, 5-H), 6,38 (d, ³*J*_{HH} = 8,0 Hz, 1H, 7-H), 3,23 (t, ³*J*_{HH} = 7,5 Hz, 2H, 2-H), 2,96 (t, ³*J*_{HH} = 7,5 Hz, 2H, 3-H), 2,84 (t, ³*J*_{HH} = 7,5 Hz, 2H, 8-H), 1,52 (q, ³*J*_{HH} = 7,5 Hz, 2H, 9-H), 1,31 (Sextett, ³*J*_{HH} = 7,5 Hz, 2H, 10-H), 0,96 (t, ³*J*_{HH} = 7,5 Hz, 3H, 11-H).
^{**13**}**C{**^{**1**}**H}-NMR** (100 MHz, 25 °C, CDCl₃): δ = 152,1 (C7a), 129,3 (C3a), 127,7 (C4), 126,6 (C6), 116,5 (C5), 106,2 (C7), 52,4 (C2), 48,3 (C8), 27,9 (C3), 22,0 (C9), 19,8 (C10), 13,3 (C11).
**MS** (70 eV): *m/z* = 223 (M⁺), 132 (M⁺ - CH₂-Ph), 117, 103, 77.

### Beispiel 8 (N-(tert-Butyl)-2,3-dihydroindol):

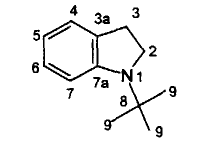

- Edukte:: 0,55 g (4,0 mmol, 0,51 ml) 3-Chlorstyrol, 0,44 g (6,0 mmol, 0,63 ml) tert-Butylamin, 10 mol% n-BuLi-Lösung (1,6 m in Hexan), 1,35 g (12,0 mmol) Kalium-tert-butylat.
- Laufmittel:: Hexan / Essigester (5:1).
- Ausbeute:: 53 % d. Th.

^{**1**}**H-NMR** (400 MHz, 25 °C, CDCl₃): δ = 6,94 (m, 2H, 4-H, 6-H), 6,53 (t, ³*J*_{HH} = 7,5 Hz, 1H, 5-H), 6,38 (d, ³*J*_{HH} = 8,0 Hz, 1H, 7-H), 3,23 (t, ³*J*_{HH} = 7,5 Hz, 2H, 2-H), 2,96 (t, ³*J*_{HH} = 7,5 Hz, 2H, 3-H), 2,84 (t, ³*J*_{HH} = 7,5 Hz, 2H, 8-H), 1,52 (q, ³*J*_{HH} = 7,5 Hz, 2H, 9-H), 1,31 (Sextett, ³*J*_{HH} = 7,5 Hz, 2H, 10-H), 0,96 (t, ³*J*_{HH} = 7,5 Hz, 3H, 11-H).
^{**13**}**C{**^{**1**}**H}-NMR** (100 MHz, 25 °C, CDCl₃): δ = 152,1 (C7a), 129,3 (C3a), 127,7 (C4), 126,6 (C6), 116,5 (C5), 106,2 (C7), 52,4 (C2), 48,3 (C8), 27,9 (C3), 22,0 (C9), 19,8 (C10), 13,3 (C11).
**MS** (70 eV): *m/z* = 176 (M⁺), 161 (M⁺ - CH₃), 120 (M⁺ - (CH₃)₂-C=CH₂), 105, 91, 77.

### c) Allgemeine Arbeitsvorschrift für die Darstellung von Indolen aus 2,3-Dihydroindolinen (im folgenden AAV3):

Nach der Darstellung der 2,3-Dihydroindoline gemäß AAV1 (allerdings mit 2,0 mmol 3-Chlorstyrol und 4,0 mmol Amin) wird jeweils die Reaktionslösung direkt weiter umgesetzt ohne das 2,3-Dihydroindolin zu isolieren. Dazu läßt man die Reaktionsmischung auf Raumtemperatur abkühlen und versetzt diese mit 2,0 mmol DDQ. Unter kräftigem Rühren läßt man die Suspension bei 120 °C Ölbadtemperatur 20 h weiterreagieren. Nach dem Abkühlen auf Raumtemperatur wird die braune Lösung mit 20 ml Wasser vermengt. Die wäßrige Phase wird dreimal mit Methylenchlorid extrahiert. Nachdem die vereinigten organischen Phasen über Magnesiumsulfat getrocknet wurden, wird das Lösemittel im Vakuum entfernt. Das so erhaltene Rohprodukt wird mit dem angegebenen Laufmittel säulenchromatographisch gereinigt.

### Beispiel 9 (N-4-Biphenylindol):

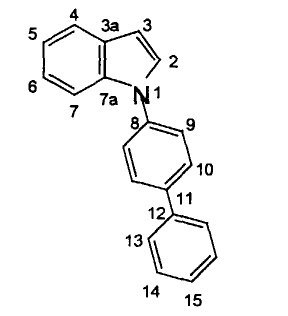

- Edukte:: 0,27 g (2,0 mmol, 0,26 ml) 3-Chlorstyrol, 0,67 g (4,0 mmol) 4-Phenylanilin, 0,67 g (6,0 mmol) Kalium-tert.-butylat, 0,45 g (2,0 mmol) DDQ.
- Laufmittel:: Hexan.
- Ausbeute:: 0,35 g ( 65 % d. Th.).

^{**1**}**H-NMR** (360 MHz, 25 °C, CDCl₃): δ = 7,63-7,26 (m, 5H, arom. H), 7,10 (m, 1H, 6-H), 7,01 (m, 8H, arom. H), 6,45 (4, ³*J*_{HH} = 8,0 Hz, 1H, 3-H).
^{**13**}**C{**^{**1**}**H}-NMR** (90 MHz, 25 °C, CDCl₃): δ = 143,1 (C-8), 137,4 (C-7a), 137,2 (C-12), 129,8 (C-3a), 129,6 (C-14), 127,5 (C-15), 127,4 (C-13), 127,3 (C-10), 126,3 (C-11), 125,0 (C-2), 122,3 (C-5), 120,5 (C-4), 120,0 (C-6), 118,2 (C-9), 110,7 (C-7), 104,1 (C-3).
**MS** (70 eV): *m/z* = 269 (M⁺-Phenyl), 134, 119, 89.

## Patentansprüche

1. Verfahren zur Herstellung von 2,3-Dihydroindolen der allgemeinen Formel (I) 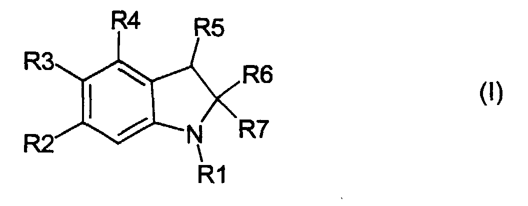 in der die Reste R1 bis R7 unabhängig voneinander aus der Gruppe von Wasserstoff, Fluor, Chlor, (C₁-C₈)-Alkyl, O-Alkyl-(C₁-C₈), OCO-Alkyl-(C₁-C₈), OH, NO₂, Si(Alkyl)₃-(C₁-C₈), CF₃, CN, COOH, CHO, SO₃H, NH₂, NH-Alkyl-(C₁-C₈), N-Alkyl₂-(C₁-C₈), NH-Ar, NAr₂, P-Alkyl₂-(C₁-C₈), SO₂-Alkyl-(C₁-C₆), SO-Alkyl-(C₁-C₆), NHCO-Alkyl-(C₁-C₄), COO-Alkyl-(C₁-C₈), CONH₂, CONH-Alkyl-(C₁-C₈), CO-Alkyl-(C₁-C₈), NHCOH, NHCOO-Alkyl-(C₁-C₄), CHCH-CO₂-Alkyl-(C₁-C₈), CHCHCO₂H, P-Alkyl₂-(C₁-C₈), POAlkyl₂-(C₁-C₄), PO₃H₂, PO(O-Alkyl-(C₁-C₆))₂, SO₃-Alkyl-(C₁-C₄), SO₂-Alkyl-(C₁-C₆), SO-Alkyl-(C₁-C₆), Si(Alkyl)₃-(C₁-C₈), Ar, O-Ar, CO-Ar, COO-Ar, PO-Ar₂ und PAr₂; wobei Ar
• einen aromatischen Rest mit bis zu 14 Kohlenstoffatomen; oder
• einen Heteroaromaten darstellt, ausgewählt aus der Gruppe der fünf-, sechs- oder siebengliedrigen Ringe mit mindestens einem Stickstoff-, Sauerstoff- und/oder Schwefelatom im Ring;
durch Umsetzung von Halogenstyrolen der allgemeinen Formel (IIa) oder (IIb) 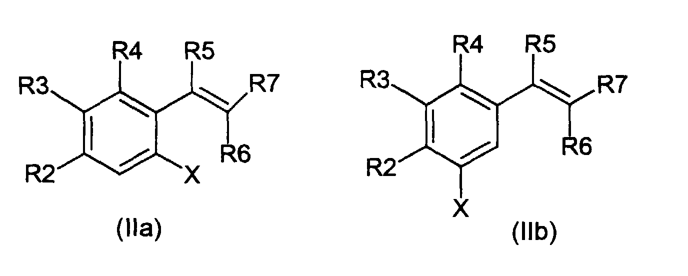 mit Aminen der allgemeinen Formel (III)
R¹-NH₂ (III)
wobei in den Formeln (IIa), (IIb) und (III)
• R¹ bis R⁷ die gleiche Bedeutung haben wie in Formel (I);
• X ausgewählt ist aus der Gruppe von Chlor, Brom, Iod, OSO₂CF₃, OSO₂Aryl-(C₆-C₁₀), OSO₂Alkyl-(C₁-C₈) und N₂⁺Y⁻, wobei Y ein Chlor-, Brom- oder lodatom oder ein Tetrafluoroborat- oder Tetraphenylboratanion darstellt;
in mindestens einem inerten Lösungsmittel oder in Wasser sowie in Gegenwart mindestens einer Base, ausgewählt aus der Gruppe von
• primären, sekundären und tertiären Alkoxiden;
• primären und sekundären Amiden von Alkali- und/oder Erdalkalielementen;
• Alkyl- und Arylverbindungen von Alkali und/oder Erdalkalimetallen; und
• Carbonaten, Hydroxiden, Hydrogencarbonaten von Lithium, Natrium, Kalium, Calcium, Magnesium und Cäsium.

2. Verfahren nach Anspruch 1, dadurch gekennzeichnet, daß der Rest R¹ einen (C₁-C₈)-Alkyl-, Phenyl-, Naphthyl-, Anthryl-, Phenanthryl-, Biphenyl-, Pyridin-, Pyrimidin-, Oxazol-, Imidazol-, Pyrazin-, Chinolin-, Indol-, Furan, Benzofuran- oder Thiophenrest darstellt.

3. Verfahren nach Anspruch 1 oder 2, dadurch gekennzeichnet, daß die Reste R² bis R⁷ unabhängig voneinander ausgewählt sind aus der Gruppe von Wasserstoff, (C₁-C₈)-Alkyl, O-Alkyl-(C₁-C₈), OCO-Alkyl-(C₁-C₈), N-Alkyl₂-(C₁-C₈), Ar, F, Cl, NO₂, CN, COOH, CHO, SO₂-Alkyl-(C₁-C₄), NH-Alkyl-(C₁-C₈), NH-Ar, NAr₂, COO-Alkyl-(C₁-C₈), CONH₂, CONH-Alkyl-(C₁-C₈), CO-Alkyl-(C₁-C₈), CO-Ar und/oder PO-Ar₂; wobei Ar die gleiche Bedeutung hat wie in Anspruch 1 und insbesondere einen Phenylrest darstellt.

4. Verfahren nach einem der vorhergehenden Ansprüche, dadurch gekennzeichnet, daß
• der aromatische Rest Ar bis zu 5 Substituenten;
• der Heteroaromat bis zu 4 Substituenten; und/oder
• der Rest R¹ bis zu 5 Substituenten
aufweisen, die unabhängig voneinander ausgewählt sind aus der Gruppe von Fluor, Chlor, CF₃, OH, NO₂, CN, R⁵, O-R⁵, CHO, CO-R⁵, COOH, COO-R⁵, OCO-R⁵, SiR⁵₃, NH₂, NH-R⁵, N-R⁵₂, SO-R⁵, SO₂-R⁵, SO₃H, SO₃-R⁵, CONH₂, NHCOH, NHCO-R⁵, NHCOO-R⁵, CHCH-CO₂-Alkyl-(C₁-C₈), PO-R⁵₂, P-R⁵₂, PO₃H₂, PO(O-Alkyl-(C₁-C₆)) und CHCHCO₂H; wobei R⁵ einen Alkylrest mit 1 bis 8 Kohlenstoffatomen oder einen Arylrest Ar darstellt; wobei Ar die gleiche Bedeutung hat wie in Anspruch 1 und insbesondere einen Phenylrest darstellt.

5. Verfahren nach einem der vorhergehenden Ansprüche, dadurch gekennzeichnet, daß R¹ einen 4-Fluorphenyl- oder 2-Methoxyphenylrest darstellt.

6. Verfahren nach einem der vorhergehenden Ansprüche, dadurch gekennzeichnet, daß der Rest Ar einen Heteroaromaten darstellt, an dessen heteroaromatischen Ring bis zu vier weitere aromatische, heteroaromatische und/oder aliphatische Ringe ankondensiert sind.

7. Verfahren nach einem der vorhergehenden Ansprüche, dadurch gekennzeichnet, daß die Base ausgewählt ist aus der Gruppe von Kaliumalkoxiden, Cäsiumalkoxiden, Alkaliamiden, Lithiumorganylen und Magnesiumorganylen.

8. Verfahren nach Anspruch 7, dadurch gekennzeichnet, daß die Base ausgewählt ist aus der Gruppe von Kalium-tert.-butanolat, Kaliumisopropanolat, Natriummethanolat, Kaliummethanolat, Natriumethanolat, Magnesiummethanolat, Calciumethanolat, Lithiumdiisopropylamid, Lithiumdiethylamid, Natriumdimethylamid, Cäsiumcarbonat, Butyllithium, Phenyllithium, Phenylmagnesiumchlorid und Kaliumhydroxid.

9. Verfahren nach einem der vorhergehenden Ansprüche, dadurch gekennzeichnet, daß das inerte Lösungsmittel ausgewählt ist aus der Gruppe von aromatischen oder aliphatischen Kohlenwasserstoffen, Estern, Aminen und Amiden.

10. Verfahren nach Anspruch 9, dadurch gekennzeichnet, daß das Lösungsmittel ausgewählt ist aus der Gruppe von THF, Dioxan, Diethylether, Diglyme, MTBE, DME, Toluol, Xylolen, Anisol, Ethylacetat, Ethylencarbonat und Propylencarbonat.

11. Verfahren nach einem der vorhergehenden Ansprüche, dadurch gekennzeichnet, daß die Base in einer Menge von 0,5 bis 10 Equivalenten, bezogen auf das Halogenstyrol der Formel (IIa) oder (IIb), eingesetzt wird.

12. Verfahren nach Anspruch 11, dadurch gekennzeichnet, daß die Base in einer Menge von 0,8 bis 5 Equivalenten, bezogen auf das Halogenstyrol der Formel (IIa) oder (IIb), eingesetzt wird.

13. Verfahren nach einem der vorhergehenden Ansprüche, dadurch gekennzeichnet, daß die Umsetzung bei einer Temperatur zwischen 0 und 200 °C, vorzugsweise zwischen 40 und 180 °C, insbesondere zwischen 60 und 160 °C, durchgeführt wird.

14. Verfahren nach einem der vorhergehenden Ansprüche, dadurch gekennzeichnet, daß die Umsetzung in Gegenwart eines zusätzlichen Dehydrierungsmittels durchgeführt wird.

15. Verfahren nach einem der Ansprüche 1 bis 13, dadurch gekennzeichnet, daß nach erfolgter Umsetzung zu der Reaktionsmischung ein Dehydrierungsmittel hinzugegeben wird.

16. Verfahren nach Anspruch 14 oder 15, dadurch gekennzeichnet, daß das Dehydrierungsmittel ausgewählt ist aus der Gruppe von Sauerstoff, Schwefel und DDQ.

17. 2,3-Dihydroindol, ausgewählt aus der Gruppe von N-(2-Methoxyphenyl)-2,3-dihydroindol, N-(4-Fluorphenyl)-4-chlor-2,3-dihydroindol, N-(4-Fluorphenyl)-2,3-dihydroindol und N-(3-Ethoxypropyl)-2,3-dihydroindol.

18. Verwendung eines Dihydroindols nach Anspruch 17 als
• Herbizid;
• Vorstufe und/oder Intermediat für die Herstellung von Farbstoffen, Aminosäuren, photosensitives und/oder thermosensitives Material, Kosmetika;
• Antioxidans für Kosmetika (im Haut- und Haarpflegebereich) und Zahnpflegeprodukte;
• Angiotensin(II)-Antagonist;
• Lipoxygenase-Inhibitor;
• Kaliumkanal-Blocker, insbesondere bei der Behandlung der Alzheimer-Krankheit oder von Depressionen;
• Inhibitor für Enzyme, insbesondere Protein-Kinase;
• Polymerisationskatalysator, insbesondere bei der Herstellung von Polyestern;
• Stabilisator für Kunststoffe, insbesondere für PVC oder Polyamide;
• Ligand für Metallkatalysatoren;
• flammenhemmendes Additiv; und
• Antistatikum in Kunststoffen.
